# EUROPÄISCHE PATENTSCHRIFT

(11) **EP 4 312 645 B1**
(45) Veröffentlichungstag und Bekanntmachung des Hinweises auf die Patenterteilung: **09.07.2025**
(21) Anmeldenummer: 22719552.6
(22) Anmeldetag: 30.03.2022
(51) Int. Cl.: A43B 7/1455, A43B 3/12, A43B 7/26, A61F 5/01

(54) **SCHUHWERK MIT EINEM ELASTISCHEN KORREKTURBAND ZUR BEHANDLUNG UND VORBEUGUNG VON FUSSFEHLSTELLUNGEN**
FOOTWEAR WITH A CORRECTING ELASTIC STRAP FOR THE TREATMENT AND PREVENTION OF FOOT DEFORMITIES
CHAUSSURES AVEC SANGLE ÉLASTIQUE CORRECTRICE POUR LE TRAITEMENT ET LA PRÉVENTION DES DÉFORMATIONS DU PIED

(30) Priorität: 30.03.2021 DE 102021108100
(43) Veröffentlichungstag der Anmeldung: 07.02.2024
(73) Patentinhaber: Hallufix AG, 82031 Grünwald (DE)
(72) Erfinder: BRASS, Manfred, 82031 Grünwald (DE); FISCHER, Franz, 92224 Amberg (DE)
(74) Vertreter: Bulat, Branimir
(86) Internationale Anmeldenummer: PCT/EP2022/058388
(87) Internationale Veröffentlichungsnummer: WO 2022/207691

(56) Entgegenhaltungen:
- CN-A- 111 387 636
- US-A1- 2011 130 695
- US-A1- 2011 179 674

## Beschreibung

### Technisches Gebiet

Die vorliegende Erfindung betrifft ein Schuhwerk zur Behandlung und Vorbeugung von Fußfehlstellungen, insbesondere zur Behandlung und Vorbeugung von Hallux valgus.

### Stand der Technik

Pathologische Fehlstellungen im Mittelfuß- und Vorfußbereich eines Patienten können unterschiedliche Ursachen haben, wie beispielsweise genetische Veranlagung, das Tragen von falschem Schuhwerk, insbesondere von zu engen oder hochhackigen Schuhen, oder eine Abflachung des Längs- und Quergewölbes infolge einer Instabilität des Bindegewebes im Mittelfußbereich. Insbesondere die als Hallux valgus bezeichnete Fehlstellung der Großzehe im Großzehengrundgelenk, auch als Valgusstellung bezeichnet, kommt hierbei aufgrund der stetig wachsenden Fallzahlen mehr Bedeutung zu.

Hallux valgus entsteht, indem die Großzehe im Großzehengrundgelenk durch Muskelzug in die fußinnenseitige Richtung gezogen wird. Dabei tritt der erste Mittelfußknochen als ballenförmige Ausbuchtung am Zehengrundgelenk fußinnenseitig hervor, was als Pseudoexostose bezeichnet wird. Zudem geht mit dem Hallux valgus häufig eine Änderung der Länge und der Zugrichtung der Sehnen einher, wodurch sich die Fehlstellung im Laufe der Zeit weiter verstärken kann. Dabei entwickelt sich eine Arthrose des Großzehengrundgelenks, die im fortgeschrittenen Stadium chirurgisch behandelt werden muss.

Um den Krankheitsprozess aufzuhalten oder diesem entgegenzuwirken, sind neben chirurgischen Eingriffen auch die Anwendung konservativer Therapiemethoden bekannt. Beispielsweise ist die Verwendung von Tapeverbänden oder Orthesen bekannt zur Behandlung des Fußes in einer Ruhestellung. Aufgrund der verlangten Ruhestellung des Fußes während der Therapie werden diese vorwiegend nachts eingesetzt.

Weiterhin ist der Einsatz von Sandalen zur therapeutischen Behandlung von Hallux valgus bekannt. EP 3 716 806 A1 offenbart beispielsweise eine Hallux-valgus-Sandale mit einem um eine zu behandelnde Zehe anzuordnendem zugstarren Schlaufenabschnitt, bei dem durch Ausübung einer Zugkraft auf den zugstarren Schlaufenabschnitt therapeutische Korrekturkräfte auf die zu behandelnde Zehe wirken.

US 2011/130695 A1 offenbart eine orthopädische Vorrichtung in der Form einer Schuheinlage mit einer Einlegesohle und einem daran befestigten Großzehenband. Weiterer Stand der Technik ist aus der US 2011/179674 A1 und CN 111 387 636 A bekannt.

### Darstellung der Erfindung

Ausgehend von dem bekannten Stand der Technik ist es eine Aufgabe der vorliegenden Erfindung, ein verbessertes Schuhwerk zur Behandlung und Vorbeugung von Fußfehlstellungen bereitzustellen, das insbesondere eine effektive therapeutische Behandlung sicherstellt und einen einfachen Aufbau aufweist.

Die Aufgabe wird durch ein Schuhwerk mit den Merkmalen des unabhängigen Anspruchs gelöst. Vorteilhafte Weiterbildungen ergeben sich aus den Unteransprüchen, der Beschreibung und den Figuren.
Entsprechend wird ein Schuhwerk zur Behandlung und Vorbeugung von Fußfehlstellungen, insbesondere zur Behandlung und Vorbeugung von Hallux valgus, bereitgestellt. Das Schuhwerk umfasst eine Sohle mit einer Laufsohle und einer Brandsohle, und ein Halteelement zum Fixieren eines Fußes relativ zu dem Schuhwerk; und ein an einer Zehe zu befestigendes Spannelement, das dazu eingerichtet ist, in einem an dem Fuß befestigten Zustand des Schuhwerks eine erste Korrekturkraft auf die Zehe auszuüben und eine der ersten Korrekturkraft entgegen gerichtete zweite Korrekturkraft auf ein Zehengrundgelenk auszuüben, wobei das Spannelement ein elastisches Korrekturband ist und wobei das Halteelement ein Halteband und ein weiteres Halteband umfasst, wobei das Halteband in einem an dem Fuß befestigten Zustand wenigstens eine zu der Zehe benachbarte weitere Zehe und/oder ein weiteres Zehengrundgelenk relativ zu dem Schuhwerk fixiert, und das weitere Halteband in dem an dem Fuß befestigten Zustand einen Mittelfußbereich des Fußes relativ zu dem Schuhwerk fixiert und im Bereich eines Spanns an dem Fuß anliegt, und wobei entgegengesetzt angeordnete Endabschnitte des Korrekturbandes, des Haltebandes und des weiteren Haltebandes in die Sohle geführt und zwischen der Laufsohle und der Brandsohle angeordnet und mit diesen form- und/oder kraft- und/oder stoffschlüssig verbunden sind.

Indem das vorgeschlagene Schuhwerk neben der auf die Zehe wirkenden ersten Korrekturkraft zusätzlich die auf das Zehengrundgelenk wirkende zweite Korrekturkraft bereitstellt, kann eine besonders effektive therapeutische Wirkung erzielt werden. Denn durch die erste und zweite Korrekturkraft kann gleichzeitig eine therapeutische Wirkung auf eine Valgusstellung der Zehe als auch auf eine Varusstellung des Zehengrundgelenks erzielt werden. Somit können Symptomatik und Ursache der Fußfehlstellung gleichzeitig behandelt werden. Die auf den Fuß wirkenden Korrekturkräfte und damit einhergehenden therapeutischen Effekte werden nachstehend im Zusammenhang mit den damit in Verbindung stehenden Komponenten des Schuhwerks genauer beschrieben.

Die vorgeschlagene Lösung zieht ferner in Betracht, dass die Zehen und das Zehengrundgelenk beim Gehen aufgrund der Beugebewegung und der auf diese wirkenden Belastungen in natürlicher Weise dazu neigen, ihre Position relativ zu dem Mittelfuß zu verändern, insbesondere in einer Fußquerrichtung. Im Rahmen der vorliegenden Erfindung wurde hierbei erkannt, dass diese relativen Bewegungen zwischen Mittelfuß, Zehe und Zehengrundgelenk Einfluss auf die therapeutische Wirkung und den Tragekomfort des Schuhwerks haben, insbesondere wenn das Schuhwerk von einem Patienten beim Gehen über einen längeren Zeitraum verwendet wird. Um diesem Umstand Rechnung zu tragen, ist das vorgeschlagene Schuhwerk mit dem als elastisches Korrekturband ausgestalteten Spannelement versehen. Das elastische Korrekturband trägt zum einen zu einem verbesserten Tragekomfort des Schuhwerks bei, indem es zu einem gewissen Grad die natürlichen Relativbewegungen zwischen den Fußkomponenten zulässt und trotzdem anhaltend die Korrekturkräfte ausübt. Darüber hinaus ermöglicht das vorgeschlagene Schuhwerk, sich diese Relativbewegungen zunutze zu machen, um die therapeutische Wirkweise des Schuhwerks zu verbessern. Denn durch die Verwendung des elastischen Korrekturbandes wird der Betrag der ausgeübten Korrekturkräfte situationsgerecht angepasst, da die durch das Korrekturband ausgeübten Kräfte von dessen elastischen Dehnung abhängen. So wird bei stark ausgeprägten Relativbewegungen auch der Betrag der Korrekturkräfte erhöht.

Gegenüber bekannten Vorrichtungen, die mit einem mehrteiligen Spannmechanismus versehen sind, der Korrekturkräfte generiert, indem manuell eine Zugkraft auf einen Zehenschlaufenabschnitt eingestellt wird, kann das vorgeschlagene Schuhwerk aufwandsreduziert am Fuß fixiert werden. Darüber hinaus kann das Schuhwerk aus weniger Komponenten bestehen und entsprechend einen kompakten und einfachen Aufbau aufweisen.

Das vorgeschlagene Schuhwerk ist dafür vorgesehen, eine pathologische Fehlstellung eines Fußes, insbesondere einer Zehe und/oder eines Zehengrundgelenks, beispielsweise eines der Zehe zugeordneten Zehengrundgelenks, zu behandeln, entgegenzuwirken und/oder vorzubeugen. Das vorgeschlagene Schuhwerk kann insbesondere zur Vorbeugung oder Behandlung von Hallux va-Igus eingesetzt werden, ist aber nicht auf diese Anwendung beschränkt.

Als Schuhwerk im Sinne der vorliegenden Offenbarung wird jedwede Art von Schuhen oder schuhartiger Fußbekleidung bezeichnet. So betrifft der Begriff "Schuhwerk" beispielsweise geschlossene Schuhe und offene Schuhe, wie zum Beispiel Sandalen, insbesondere Zehenstegsandalen, und dergleichen. Entsprechend ist das vorgeschlagene Schuhwerk dafür vorgesehen, an einem Fuß eines Patienten befestigt zu werden und in dem am Fuß befestigten Zustand auf den Fuß, insbesondere auf die Zehe und das Zehengrundgelenk therapeutisch einzuwirken. Hierbei betrifft der Begriff "Schuhwerk" sowohl ein Paar von Schuhen, d. h. ein Paar bestehend aus einem linken Schuh und einem rechten Schuh, als auch nur einen einzelnen Schuh.

In der vorliegenden Offenbarung bezieht sich der Ausdruck "in einem/dem am Fuß ordnungsgemäß befestigten Zustand", vorliegend auch mit "in dem befestigten Zustand" abgekürzt, auf einen Zustand, in dem das Schuhwerk an einem Fuß eines Patienten bestimmungsgemäß befestigt ist und entsprechend einen gewünschten therapeutischen Effekt zur Korrektur oder zur Vorbeugung von Fehlstellungen bewirkt.

Das vorgeschlagene Schuhwerk ist derart ausgestaltet, dass es in dem befestigten Zustand Korrekturkräfte auf den Fuß ausübt. Unter dem Begriff "Korrekturkräfte" werden vorliegend solche Kräfte verstanden, die eine therapeutische Wirkung auf den zu behandelnden Fuß haben. Insbesondere bewirken die Korrekturkräfte, dass die von der Fehlstellung betroffenen Teile des Fußes in eine anatomisch korrekte oder beabsichtigte Position gelenkt werden, um einen gewünschten therapeutischen Effekt zu erzielen.

Wir vorrangehend beschrieben wurde im Rahmen der vorliegenden Offenbarung erkannt, dass eine besonders effektive therapeutische Wirkung erzielt werden kann, indem das Schuhwerk neben der auf die Zehe wirkenden ersten Korrekturkraft zusätzlich die auf das Zehengrundgelenk wirkende zweite Korrekturkraft ausübt. Im Zusammenhang mit der vorliegenden Offenbarung betrifft das Merkmal, dass die "zweite Korrekturkraft auf das Zehengrundgelenk ausgeübt wird", solche Korrekturkräfte, die auf das Zehengrundgelenk therapeutisch einwirken. Solche Korrekturkräfte können mittels des Korrekturbandes unmittelbar in das Zehengrundgelenk eingeleitet werden oder unmittelbar in einen seitlich ausbuchtenden Teil des Zehenballens, insbesondere eine Pseudoexostose. Alternativ können solche Korrekturkräfte mittels des Korrekturbandes mittelbar auf das Zehengrundgelenk oder den ausbuchtenden Teil des Zehenballens einwirken, beispielsweise indem eine Korrekturkraft auf einen Mittelfußknochen, insbesondere den ersten Mittelfußknochen, im Bereich des Zehengrundgelenks ausgeübt wird. Die zweite Korrekturkraft hat insbesondere den Zweck, auf die Varusstellung eines Mittelfußknochens, im Speziellen auf die Varusstellung des ersten Mittelfußknochens, therapeutisch einzuwirken. Das dadurch resultierende Zusammenspiel von auf den Fuß ausgeübten Korrekturkräften kann besonders vorteilhaft bei der Behandlung von Hallux valgus sein. Wie vorangehend weiterhin beschrieben, ist die zweite Korrekturkraft entgegen der ersten Korrekturkraft gerichtet. Hierunter wird in der vorliegenden Offenbarung verstanden, dass ein die erste Korrekturkraft darstellender Vektor entgegengesetzt zu einem die zweite Korrekturkraft darstellenden Vektor ist. Die die erste und die zweite Korrekturkraft darstellenden Vektoren sind hierbei parallel zueinander, zeigen aber in unterschiedliche Richtungen und sind insbesondere beabstandet zueinander.

Im Zusammenhang mit dem Korrekturband wird nachfolgend zur Vereinfachung allgemein auf eine Zehe des Fußes Bezug genommen. Hierbei kann es sich um die Großzehe des zu behandelnden Fußes handeln. Das Schuhwerk ist hierauf aber nicht beschränkt, sodass mit dem Ausdruck beispielsweise auch die Kleinzehe gemeint sein kann. Im Zusammenhang mit der zweiten Korrekturkraft wird entsprechend zur Vereinfachung allgemein auf ein Zehengrundgelenk Bezug genommen, wobei insbesondere das Großzehengrundgelenk gemeint sein kann, das Schuhwerk hierauf aber nicht beschränkt ist. Alternativ kann es sich beispielsweise auch um das Kleinzehengrundgelenk handeln.

In der vorliegenden Offenbarung wird zur Beschreibung des Schuhwerks, insbesondere in Bezug auf den zu behandelnden Fuß, ein Bezugssystem verwendet, das auf die Mittellinie des Körpers eines Patienten ausgerichtet ist, wie in der Anatomie üblich. So können die Position und Richtung der einzelnen Komponenten des vorgeschlagenen Schuhwerks in dem befestigten Zustand in Bezug auf den in dem Schuhwerk aufgenommenen Fuß angegeben werden. Dementsprechend bezieht sich der Begriff "medial" auf eine Richtung oder Seite des Schuhwerks, die in Richtung einer medialen Ebene des Körpers des Trägers zeigt. In der Anatomie bezieht sich der Begriff "mediale Ebene", die auch als "Mittelsagittalebene" bezeichnet wird, im Allgemeinen auf eine anatomische Ebene, die den Körper in zwei symmetrische Teile teilt. Dementsprechend bedeutet der Begriff "in medialer Richtung" bei der Beschreibung eines Schuhwerks eine Richtung, die von dem zu behandelnden Fuß des Patienten in Richtung seines anderen Fußes zeigt. In diesem Sinne bezieht sich der Begriff "lateral" auf eine Richtung oder Seite des Schuhwerks, die von der medialen Ebene des Körpers des Trägers weg weist. Dementsprechend bedeutet der Begriff "in lateraler Richtung" bei der Beschreibung des Schuhwerks, welche an einem Fuß des Trägers befestigt ist, in einer Richtung, die von dem anderen Fuß des Trägers weg weist.

Zum Bereitstellen der Korrekturkraft ist das vorgeschlagene Schuhwerk mit dem als elastisches Korrekturband ausgebildeten Spannelement versehen. Unter dem Begriff "elastisches Band", insbesondre "elastisches Korrekturband" und "elastisches Halteband", wird in der vorliegenden Offenbarung eine band- oder riemenförmige Komponente verstanden, die zu einem nicht unwesentlichen Betrag elastisch verformbar ist, um die erste und zweite Korrekturkraft zu generieren. Dies ist ein wesentlicher Unterschied zu einem zugstarren Band. In der hier vorliegenden Anwendung sind jedenfalls solche Komponenten als elastisch zu betrachten, die für Belastungen von wenigstens 10 % des Betrags der ersten oder zweiten Korrekturkraft ein lineares oder nicht-lineares elastisches Verhalten zeigen. Mit anderen Worten kann das Korrekturband jedenfalls wenigstens 10 %, insbesondere im Wesentlichen 100 % des Betrags der ersten oder zweiten Korrekturkraft in Form von durch eine elastische Verformung bewirkten Spannkräften aufnehmen. Mit anderen Worten, die durch die elastische Verformung bewirkten Spannkräfte in dem Korrekturband können wenigstens 10 %, insbesondere im Wesentlichen 100 % des Betrags der ersten oder zweiten Korrekturkraft betragen. Das Korrekturband ist insbesondere entlang dessen Längsrichtung und optional entlang dessen Querrichtung elastisch verformbar.

Das vorgeschlagene Schuhwerk ist insbesondere derart bereitgestellt, dass in dem an dem Fuß befestigten Zustand das Korrekturband elastisch verformt ist. Mit anderen Worten kann das Korrekturband in dem an dem Fuß befestigten Zustand in einem Spannzustand angeordnet sein, in dem es elastisch verformt ist und entsprechend durch die elastische Verformung bewirkten Spannkräften unterworfen ist. Ist das Schuhwerk hingegen von dem zu behandelnden Fuß entkoppelt, kann sich das Korrekturband in einem Ruhezustand befinden. In dem an dem Fuß befestigten Zustand, d. h. wenn das Korrekturband in dem Spannzustand angeordnet ist, kann das Korrekturband wenigstens 2 mm oder wenigstens 3 mm oder wenigstens 5 mm elastisch verformt sein. Mit anderen Worten kann in dem am Fuß befestigten Zustand, d. h. wenn das Korrekturband in dem Spannzustand angeordnet ist, eine Länge des Korrekturbandes um wenigstens 2 mm oder wenigstens 3 mm oder wenigstens 5 mm durch elastische Verformung verlängert sein gegenüber dem Ruhezustand des Korrekturbandes. Entsprechend kann das Schuhwerk derart ausgebildet und eingerichtet sein, dass in dem befestigten Zustand die erste Korrekturkraft und/oder die zweite Korrekturkraft zumindest teilweise erzeugt sind/ist durch eine in dem Korrekturband vorherrschende und durch elastische Verformung bewirkte Spannkraft.

Das Schuhwerk ist vorzugsweise derart eingerichtet, dass in dem an dem Fuß befestigten Zustand das Korrekturband zumindest abschnittsweise an dem Fuß anliegt, insbesondere im Bereich der Zehe und/oder im Bereich des Zehengrundgelenks, insbesondere im Bereich des seitlich ausbuchtenden Teil des Zehenballens. Die an der Zehe und/oder dem Zehengrundgelenk, insbesondere im Bereich des seitlich ausbuchtenden Teils des Zehenballens, anliegende Fläche des Korrekturbandes wird vorliegend als Wirkfläche bezeichnet. Das Schuhwerk kann derart ausgebildet sein, dass in dem an dem Fuß befestigten Zustand das Korrekturband entlang seiner Wirkfläche, insbesondere entlang der gesamten Wirkfläche oder abschnittsweise, und/oder entlang zu der Wirkfläche angrenzenden Abschnitten elastisch verformt ist.

Das Korrekturband kann mehrschichtig aufgebaut sein. Beispielsweise kann das Korrekturband eine elastisch verformbare Schicht aufweisen, die wesentlich zu der elastischen Eigenschaft des Korrekturbandes beitragen kann. Die elastisch verformbare Schicht kann durch ein Gummimaterial bereitgestellt sein, beispielsweise durch einen Elastomer. Alternativ oder zusätzlich kann das Korrekturband wenigstens eine Auflageschicht aufweisen. Eine erste Auflageschicht kann entlang der Wirkfläche angeordnet sein. Die erste Auflageschicht kann beispielsweise aus einem Vlies hergestellt sein. Auf diese Weise kann der Tragekomfort erhöht werden. Gemäß einer Ausführungsform kann das Korrekturband derart ausgestaltet sein, dass die erste Auflageschicht und eine entsprechend ausgestaltete zweite Auflageschicht an entgegengesetzten Seiten der elastisch verformbaren Schicht angeordnet sein können.

In dem befestigten Zustand kann das Korrekturband die Zehe zumindest abschnittsweise umgreifen. Unter einem Umgreifen der Zehe wird vorliegend verstanden, dass das Korrekturband sich in dem am Fuß befestigten Zustand in Zehenumfangsrichtung entlang der Zehe erstreckt. Vorzugsweise erstreckt sich das Zehensegment um die Zehe oder um die Zehe und das Zehengrundgelenk über ein Bogenmaß von im Wesentlichen π rad um die Zehenlängsachse. Das bedeutet, dass sich das Korrekturband in Umfangsrichtung der Zehe wenigstens entlang einer Hälfte des Umfangs einer Zehe erstreckt. Mit anderen Worten kann sich das Korrekturband von einer Seite der Zehe zu der entgegengesetzten Seite der Zehe oder des Zehengrundgelenks erstrecken. Gemäß einer Ausführungsform kann sich das Korrekturband von einer lateralen Seite der Zehe entlang einer Zehenoberfläche zu einer medialen Seite des Fußes im Bereich des Zehengrundgelenks oder eines Zehenballens erstrecken. Alternativ kann sich das Korrekturband von einer medialen Seite der Zehe, insbesondere einer Kleinzehe, entlang einer Zehenoberfläche zu einer lateralen Seite des Fußes im Bereich des Zehengrundgelenks, insbesondere des Kleinzehengrundgelenks erstrecken. Mit anderen Worten kann die Wirkfläche in der Art einer Wendefläche ausgebildet sein, deren Flächennormal sich entlang einer Zehengrundachse ändert, aber insbesondere im Wesentlichen auf die Zehengrundachse zeigt.

Das Korrekturband umfasst entgegengesetzt angeordnete Endabschnitte, insbesondere einen ersten Endabschnitt und einen dazu entgegengesetzt angeordneten zweiten Endabschnitt. Das Schuhwerk kann derart ausgestaltet sein, dass in dem an dem Fuß befestigten Zustand der erste Endabschnitt zwischen der Zehe und einer dazu benachbarten weiteren Zehe, insbesondere der zweiten Zehe des Fußes, positioniert ist und insbesondere in eine Sohle des Schuhwerks geführt ist. Der zweite Endabschnitt kann im Bereich des Zehengrundgelenks positioniert sein und in die Sohle geführt sein. Der zweite Endabschnitt kann seitlich an dem Zehengrundgelenk oder dem seitlich ausbuchtenden Teils des dem Zehengrundgelenk zugeordneten Zehenballens anliegen und insbesondere abschnittsweise umgreifen. Alternativ kann der zweite Endabschnitt in einer von dem Zehengrundgelenk zu der Zehe zeigenden Richtung vor oder nach dem Zehengrundgelenk angeordnet sein.

Weiterhin ist das Korrekturband über den ersten Endabschnitt und den zweiten Endabschnitt mit der Sohle verbunden, insbesondere fest, im Speziellen zugfest, verbunden. Hierzu sind der erste Endabschnitt und der zweite Endabschnitt formschlüssig, kraftschlüssig und/oder stoffschlüssig mit der Sohle verbunden.

Die Sohle des Schuhwerks ist mehrschichtig aufgebaut. Mit anderen Worten kann die Sohle des Schuhwerks aus mehreren flachen Schichten aufgebaut sein. Die Sohle umfasst eine Laufsohle und eine Brandsohle, wobei der erste Endabschnitt und der zweite Endabschnitt abschnittsweise zwischen der Laufsohle und der Brandsohle angeordnet sind.

In einer Weiterentwicklung kann ein Verbindungsabschnitt des Korrekturbandes, über den der erste Endabschnitt des Korrekturbandes mit der Sohle verbunden ist, beabstandet angeordnet sein zu einem proximalen Ende eines Zehenzwischenraums zwischen der Zehe und der benachbarten weiteren Zehe. Ein Abstand zwischen dem Verbindungsabschnitt und dem proximalen Ende des Zehenzwischenraums entlang einer Fußlängsachse kann in einem Bereich zwischen 4 mm und 18 mm liegen, insbesondere in einem Bereich zwischen 6 mm und 15 mm.

Das Schuhwerk umfasst ferner das Halteelement, das dazu eingerichtet ist, in dem befestigten Zustand den zu behandelnden Fuß relativ zu dem Schuhwerk zu fixieren, insbesondere in einer vordefinierten Position zu halten. Mit anderen Worten kann das Halteelement dafür vorgesehen sein, in dem an dem Fuß befestigten Zustand eine Haltekraft auf den Fuß auszuüben. Durch das Zusammenwirken der Korrekturkräfte und der Haltekraft kann das Schuhwerk zuverlässig an dem zu behandelnden Fuß in einer für die therapeutische Behandlung vorgesehenen Position stabil gehalten werden und gleichzeitig auf den Fuß therapeutisch einwirken.

Das Halteelement umfasst das Halteband, das dazu eingerichtet ist, in dem an dem Fuß befestigten Zustand wenigstens eine zu der Zehe benachbarte weitere Zehe, insbesondere die zweite Zehe oder die zweite bis fünfte Zehe, und/oder ein zu dem Zehengrundgelenk entgegengesetzt angeordnetes weiteres Zehengrundgelenk, insbesondere das Kleinzehengrundgelenk, relativ zu dem Schuhwerk zu fixieren. Im Speziellen kann das Halteband eine Haltekraft auf die zu der Zehe benachbarte weitere Zehe ausüben, wobei die Haltekraft insbesondere der ersten Korrekturkraft entgegen gerichtet sein kann. Auf diese Weise stellt das Halteband sicher, dass die Korrekturkräfte auf die Zehe und das Zehengrundgelenk ordnungsgemäß wirken können, da durch die Haltekraft der Fuß stabil in dem Schuhwerk gehalten werden kann.

In einer Weiterentwicklung kann die durch das Halteband auszuübende Haltekraft eine therapeutische Wirkung auf den zu behandelnden Fuß haben, die insbesondere zur therapeutischen Wirkung der ersten und zweiten Korrekturkraft beiträgt und/oder eine davon zu unterscheidende, weitere therapeutische Wirkung bereitstellt.

Das Halteband kann in dem an dem Fuß befestigten Zustand an der zu der Zehe benachbarten weiteren Zehe anliegen und/oder im Bereich des zu dem Zehengrundgelenk entgegengesetzt angeordneten weiteren Zehengrundgelenks am Fuß anliegen. In einer Weiterentwicklung kann in dem am Fuß befestigten Zustand das Halteband derart an dem Fuß anliegen, dass sich das Halteband von einer Seite der weiteren Zehe, insbesondere der zweiten Zehe, zu einer entgegengesetzten Seite des weiteren Zehengrundgelenks erstreckt. Im Speziellen kann sich das Halteband von einer medialen Seite der weiteren Zehe, insbesondere der zweiten Zehe, zu lateralen Seite des weiteren Zehengrundgelenks erstrecken.

Das Halteband kann insbesondere ein elastisches Halteband sein, das in dem an dem Fuß befestigten Zustand elastisch verformt sein kann. Das Halteband ist insbesondere entlang dessen Längsrichtung und optional entlang dessen Querrichtung elastisch verformbar. Mit anderen Worten kann in dem an dem Fuß befestigten Zustand das Halteband in einem Spannzustand angeordnet sein, in dem es elastisch verformt ist und entsprechend durch die elastische Verformung bewirkten Spannkräften unterworfen ist. Ist das Schuhwerk hingegen von dem zu behandelnden Fuß entkoppelt, befindet sich das Halteband in einem Ruhezustand. In dem am Fuß befestigten Zustand, d. h. wenn das weitete Halteband in dem Spannzustand angeordnet ist, kann das Halteband wenigstens 2 mm oder wenigstens 3 mm oder wenigstens 5 mm elastisch verformt sein. Mit anderen Worten kann in dem an dem Fuß befestigten Zustand, d. h. wenn das Halteband in dem Spannzustand angeordnet ist, eine Länge des Haltebandes um wenigstens 2 mm oder wenigstens 3 mm oder wenigstens 5 mm durch elastische Verformung verlängert sein gegenüber dem Ruhezustand des Haltebandes. Entsprechend kann das Schuhwerk derart ausgebildet und eingerichtet sein, dass in dem befestigten Zustand die durch das Halteband ausgeübte Haltekraft zumindest teilweise erzeugt ist durch eine in dem Halteband vorherrschende und durch elastische Verformung bewirkte Spannkraft. Bezogen auf seine Materialeigenschaften, insbesondere im Hinblick auf dessen Elastizitätseigenschaften, kann das Halteband entsprechend zu dem Korrekturband ausgestaltet sein. Mit anderen Worten kann das Halteband aus demselben Material wie das Korrekturband hergestellt sein.

Das Halteelement umfasst ein weiteres Halteband , das in dem an dem Fuß befestigten Zustand einen Mittelfußbereich des Fußes relativ zu dem Schuhwerk fixiert und im Bereich eines Spanns oder Rists an dem Fuß anliegt. Das weitere Halteband kann aus einem im Wesentlichen zugstarren Material ausgebildet sein. Mit anderen Worten kann das weitere Halteband ein zugstarres Haltebandsein.

Das weitere Halteband kann einen distalen Endabschnitt und zwei proximale Endabschnitte umfassen, die fest, insbesondere zugstarr, mit der Sohle verbunden sein können. Das Schuhwerk kann derart ausgestaltet sein, dass in dem an dem Fuß befestigten Zustand der distale Endabschnitt zwischen der Zehe und der dazu benachbarten weiteren Zehe positioniert und an der Sohle befestigt ist und die zwei proximalen Endabschnitte an entgegengesetzten Seiten, d. h. an einer lateralen und einer medialen Seite, an dem Fuß positioniert und an der Sohle befestigt sind.

### Kurze Beschreibung der Figuren

Bevorzugte weitere Ausführungsformen der Erfindung werden durch die nachfolgende Beschreibung der Figuren näher erläutert. Dabei zeigen schematisch:
- Figur 1: eine Draufsicht auf ein Schuhwerk zur Behandlung und Vorbeugung von Fußfehlstellungen in einem an einem Fuß eines Patienten befestigten Zustand; und
- Figur 2 und 3: das in Figur 1 gezeigte Schuhwerk in perspektivischen Ansichten von unterschiedlichen Seiten.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

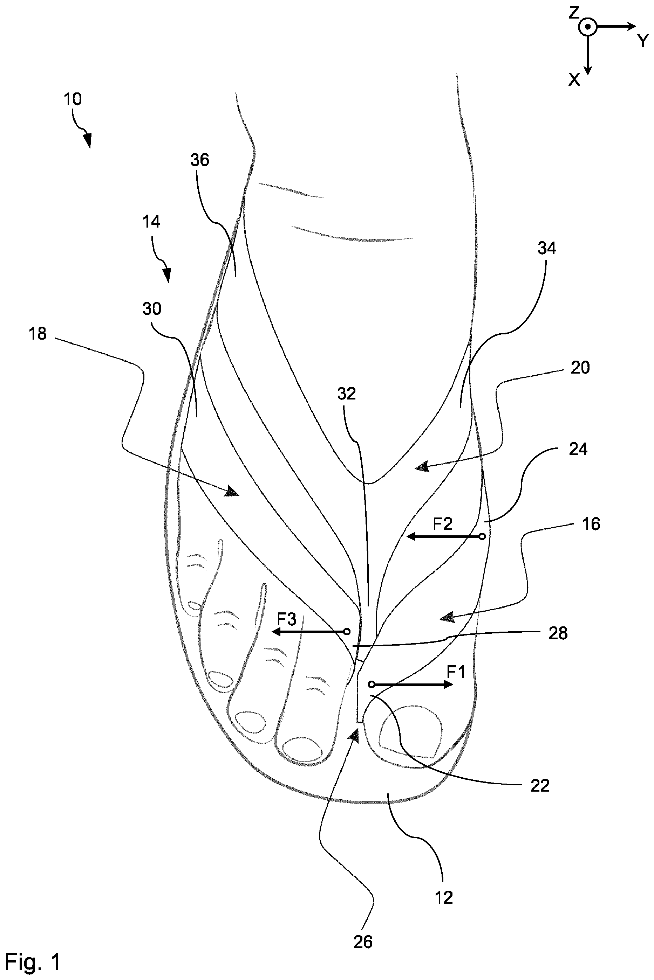

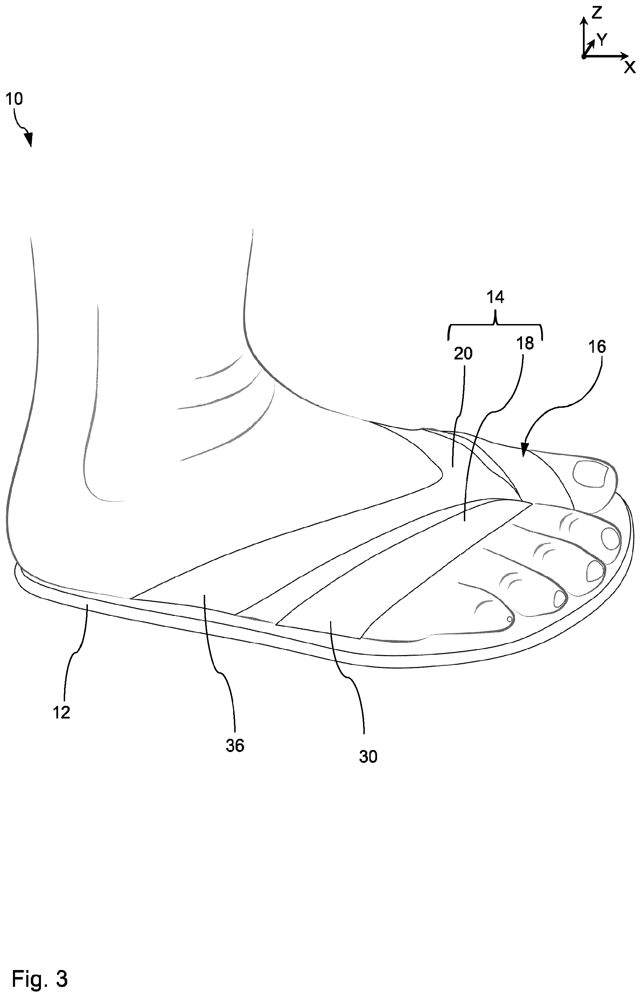

Figur 1 bis 3 zeigen ein Schuhwerk 10 zur Behandlung und Vorbeugung von Fußfehlstellungen, insbesondere zur Behandlung und Vorbeugung von Hallux valgus. Das Schuhwerk 10 ist in Form einer Sandale bereitgestellt, insbesondere in der Art einer Zehenstegsandale.

Das Schuhwerk 10 umfasst eine Sohle 12, an der ein Halteelement 14 und ein Spannelement 16 befestigt sind, die jeweils Schlaufen zur Aufnahme unterschiedlicher Abschnitte des zu behandelnden Fußes bilden.

Das Spannelement 16 ist derart ausgebildet, dass dieses eine Schlaufe bildet, durch die eine Großzehe des Fußes, nachfolgend als "Zehe" bezeichnet, geführt ist. Das Spannelement 16 ist dazu eingerichtet, an der Zehe befestigt zu werden und in dem an dem Fuß befestigten Zustand, der in Figur 1 gezeigt ist, eine erste Korrekturkraft F1 auf die Zehe auszuüben und eine der ersten Korrekturkraft entgegen gerichtete zweite Korrekturkraft F2 auf ein Zehengrundgelenk auszuüben, wie in Figur 1 durch die Korrekturkräfte abzweigende Vektoren "F1" und "F2" angedeutet. Das Spannelement 16 ist hierbei in der Form eines elastischen Korrekturbandes, nachfolgend als "Korrekturband" bezeichnet, bereitgestellt.

Das Halteelement 14 ist zum Fixieren des Fußes relativ zu dem Schuhwerk 10, insbesondere relativ zu der Sohle 12, vorgesehen und umfasst hierzu ein erstes Halteband 18 und ein separates zweites Halteband 20. Das erste Halteband 18 ist derart ausgebildet, dass dieses eine Schlaufe bildet, durch die eine zweite bis fünfte Zehe des Fußes geführt sind, wie in Figur 1 gezeigt. Das erste Halteband 18 ist hierbei in der Form eines elastischen Haltebandes bereitgestellt.

Das zweite Halteband 20 ist dazu eingerichtet, in dem an dem Fuß befestigten Zustand einen Mittelfußbereich des Fußes relativ zu dem Schuhwerk 10 zu fixieren. Hierzu liegt das zweite Halteband 20 im Bereich eines Spanns oder Rist an dem Fuß an, wie in Figuren 1 bis 3 gezeigt. Das zweite Halteband 20 ist hierbei in der Form eines zugstarren Haltebandes bereitgestellt.

Die Sohle 12 des Schuhwerks 10 ist mehrschichtig aufgebaut und umfasst eine Laufsohle und eine Brandsohle. Das Korrekturband 16, das erste Halteband 18 und das zweite Halteband 20 sind fest mit der Sohle 12 verbunden. Hierzu sind entgegengesetzt angeordnete Endabschnitte des Korrekturbandes 16, des ersten Haltebandes 18 und des zweiten Haltebandes 20 fest und zugstarr mit der Sohle 12 verbunden. Hierzu sind die entgegengesetzt angeordneten Endabschnitte des Korrekturbandes 16, des ersten Haltebandes 18 und des zweiten Haltebandes 20 in die Sohle geführt und zwischen der Laufsohle und der Brandsohle angeordnet und mit diesen form- und/oder kraft- und/oder stoffschlüssig verbunden.

Das Korrekturband 16 und das erste Halteband 18 sind in dem an dem Fuß befestigten Zustand elastisch verformt, insbesondere um wenigstens 3 mm elastisch verformt. Mit anderen Worten sind das Korrekturband 16 und das erste Halteband 18 in dem an dem Fuß befestigten Zustand in einem Spannzustand angeordnet, in dem diese elastisch verformt sind und entsprechend durch die elastische Verformung bewirkten Spannkräften unterworfen sind. Ist das Schuhwerk 10 hingegen von dem zu behandelnden Fuß entkoppelt, sind das Korrekturband 16 und das erste Halteband 18 in einem Ruhezustand angeordnet. In dem an dem Fuß befestigten Zustand, d. h. wenn das Korrekturband 16 und das erste Halteband 18 in dem Spannzustand angeordnet sind, sind das Korrekturband und das erste Halteband wenigstens 2 mm oder wenigstens 3 mm oder wenigstens 5 mm elastisch verformt. Mit anderen Worten ist in dem am Fuß befestigten Zustand eine Länge, insbesondere Umschlingungslänge, des Korrekturbandes 16 und des ersten Haltebandes 18 um wenigstens 2 mm oder wenigstens 3 mm oder wenigstens 5 mm durch elastische Verformung verlängert gegenüber deren Ruhezustand. In dem befestigten Zustand sind die durch das Korrekturband 18 ausgeübte erste und zweite Korrekturkraft und eine durch das erste Halteband ausgeübte Haltekraft F3 erzeugt durch eine in dem Korrekturband und dem ersten Halteband 18 vorherrschende und durch elastische Verformung bewirkte Spannkraft. Die Haltekraft F3 wird hierbei auf die zweite Zehe ausgeübt in einer zu der ersten Korrekturkraft entgegengesetzten Richtung, wie in Figur 1 gezeigt.

Das Korrekturband und das erste Halteband sind mehrschichtig aufgebaut. Genauer umfassen diese eine elastische Schicht, insbesondere aus einem Elastomer, die von zwei Auflageschichten an entgegengesetzten Flächen abgedeckt ist. Die Auflageschichten sind aus einem Vliesmaterial hergestellt.

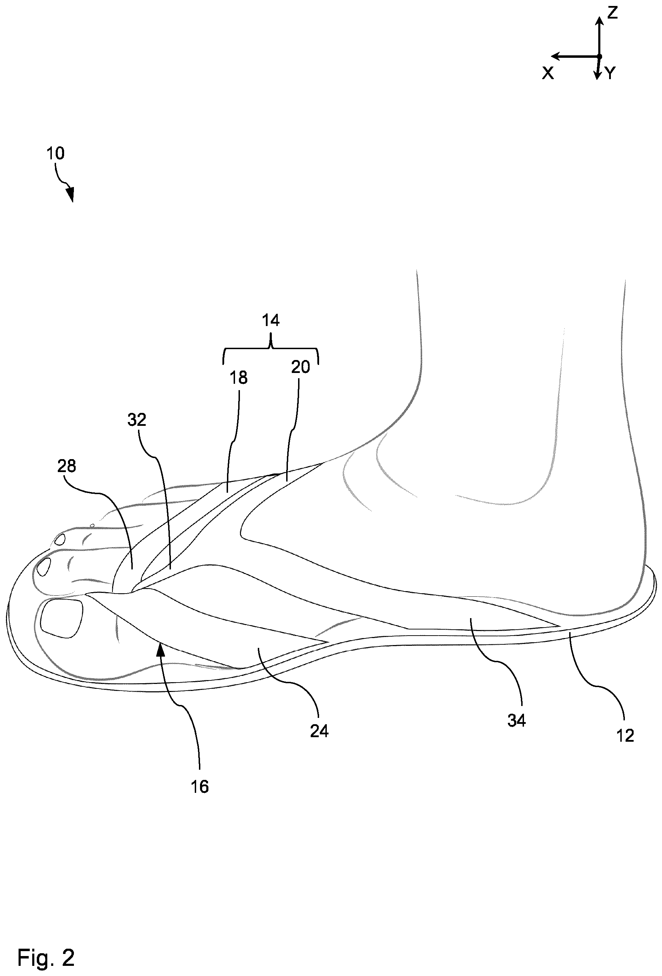

Das Korrekturband 16 liegt entlang einer Wirkfläche an der Zehe und dem Zehengrundgelenk an. In dem an dem Fuß befestigten Zustand ist das Korrekturband 16 entlang seiner Wirkfläche und/oder entlang zu der Wirkfläche angrenzenden Abschnitten elastisch verformt. In dem Spannzustand umgreift das Korrekturband 16 die Zehe zumindest abschnittsweise derart, dass das Korrekturband sich von einer lateralen Seite der Zehe entlang einer Zehenoberfläche zu einer medialen Seite des Fußes im Bereich des Zehengrundgelenks erstreckt, wie in Figur 1 und 2 gezeigt.

Das Korrekturband umfasst einen ersten Endabschnitt 22 und einen dazu entgegengesetzt angeordneten zweiten Endabschnitt 24. In dem am Fuß befestigten Zustand ist der erste Endabschnitt 22 zwischen der Großzehe und der zweiten Zehe positioniert, genauer in einem Zwischenraum zwischen diesen. Der zweite Endabschnitt 24 ist im Bereich des Großzehengrundgelenks positioniert, wie in Figur 1 gezeigt. Das Korrekturband 16 ist hierbei über den ersten Endabschnitt 22 und den zweiten Endabschnitt 24 mit der Sohle 12 des Schuhwerks 10 zugstarr verbunden, wie vorangehend bereits beschrieben. Der zweite Endabschnitt 24 liegt seitlich an dem Zehengrundgelenk, d.h. an dem seitlich ausbuchtenden Teil des Zehenballens an. Alternativ kann der zweite Endabschnitt 24 in einer von dem Zehengrundgelenk zu der Zehe zeigenden Richtung, d.h. entlang der Längsrichtung X, vor oder nach dem Zehengrundgelenk oder dem Zehenballen angeordnet sein.

Der erste Endabschnitt 22 des Korrekturbandes 16 ist über einen Verbindungsabschnitt 26 mit der Sohle 12 verbunden. Der Verbindungsabschnitt 26 bildet denjenigen Abschnitt, an dem das Korrekturband 16 mit der Sohle 12 in Kontakt tritt bzw. in die Sohle 12 geführt wird. Mit anderen Worten steht der erste Endabschnitt 22 an dem Verbindungabschnitt 26 von der Sohle 12 ab. Der Verbindungsabschnitt 26 ist beabstandet zu einem proximalen Ende des Zehenzwischenraums angeordnet und zwar derart, dass ein Abstand zwischen dem Verbindungsabschnitt 26 und dem proximalen Ende des Zehenzwischenraums entlang der Fußlängsachse X in einem Bereich zwischen 6 mm und 16 mm liegt.

Das erste Halteband 18 ist dazu eingerichtet, in dem an dem Fuß befestigten Zustand wenigstens die zweite Zehe und das Kleinzehengrundgelenk relativ zu dem Schuhwerk 10 zu fixieren. Hierzu liegt das erste Halteband 18 an der zweiten Zehe und dem Kleinzehengrundgelenk an. Genauer umfasst das erste Halteband 18 einen ersten Endabschnitt 28 und einen dazu entgegengesetzt angeordneten zweiten Endabschnitt 30. In dem am Fuß befestigten Zustand ist der erste Endabschnitt 28 zwischen der Großzehe und der zweiten Zehe positioniert, genauer in dem Zwischenraum zwischen diesen. Der zweite Endabschnitt 30 ist im Bereich des Kleinzehengrundgelenks positioniert, wie in Figur 1 gezeigt. Das erste Halteband 18 ist hierbei über dessen ersten Endabschnitt 28 und dessen zweiten Endabschnitt 30 mit der Sohle 12 des Schuhwerks 10 zugstarr verbunden, wie vorangehend bereits beschrieben. In der hier gezeigten Konfiguration liegt der zweite Endabschnitt 30 derart an dem Fuß an, dass dieser in der Längsrichtung X vor dem Kleinzehengrundgelenk angeordnet ist. Alternativ kann der zweite Endabschnitt 30 in der Längsrichtung X hinter dem Kleinzehengrundgelenk angeordnet sein oder an diesem seitlich anliegen.

Das im Wesentlichen zugstarre zweite Halteband 20 umfasst einen distalen Endabschnitt 32 und zwei proximale Endabschnitte 34, 36, die zugstarr mit der Sohle 12 verbunden sind. Der distale Endabschnitt 32 ist in dem Zehenzwischenraum zwischen der Großzehe und der zweiten Zehe angeordnet und liegt an dem proximalen Ende des Zehenzwischenraums an. Die zwei proximalen Endabschnitte 34, 36 sind an entgegengesetzten Seiten, genauer an einer lateralen und einer medialen Seite, an dem Fuß positioniert und an der Sohle befestigt.

### Bezugszeichenliste

- 10: Schuhwerk
- 12: Sohle
- 14: Halteelement
- 16: Spannelement; Korrekturband
- 18: erstes Halteband
- 20: zweites Halteband
- 22: erster Endabschnitt des Korrekturbandes
- 24: zweiter Endabschnitt des Korrekturbandes
- 26: Verbindungsabschnitt
- 28: erster Endabschnitt des ersten Haltebandes
- 30: zweiter Endabschnitt des ersten Haltebandes
- 32: proximaler Endabschnitt des zweiten Haltebandes
- 34,36: distale Endabschnitte des zweiten Haltebandes
- F1: erste Korrekturkraft
- F2: zweite Korrekturkraft
- F3: Haltekraft

## Patentansprüche

1. Schuhwerk (10) zur Behandlung und Vorbeugung von Fußfehlstellungen, insbesondere zur Behandlung und Vorbeugung von Hallux valgus, umfassend
- eine Sohle (12) mit einer Laufsohle und einer Brandsohle;
- ein Halteelement (14) zum Fixieren eines Fußes relativ zu dem Schuhwerk (10); und
- ein an einer Zehe zu befestigendes Spannelement (16), das dazu eingerichtet ist, in einem an dem Fuß befestigten Zustand des Schuhwerks (10) eine erste Korrekturkraft (F1) auf die Zehe auszuüben und eine der ersten Korrekturkraft (F1) entgegen gerichtete zweite Korrekturkraft (F2) auf ein Zehengrundgelenk auszuüben, wobei
das Spannelement (16) ein elastisches Korrekturband ist, wobei
das Halteelement (14) ein Halteband (18) und ein weiteres Halteband (20) umfasst, wobei
das Halteband (18) in einem an dem Fuß befestigten Zustand wenigstens eine zu der Zehe benachbarte weitere Zehe und/oder ein weiteres Zehengrundgelenk relativ zu dem Schuhwerk (10) fixiert, und
das weitere Halteband (20) in dem an dem Fuß befestigten Zustand einen Mittelfußbereich des Fußes relativ zu dem Schuhwerk (10) fixiert und im Bereich eines Spanns an dem Fuß anliegt, und wobei entgegengesetzt angeordnete Endabschnitte (22, 24, 32, 34, 36) des Korrekturbandes (16), des Haltebandes (18) und des weiteren Haltebandes (20) in die Sohle (12) geführt und zwischen der Laufsohle und der Brandsohle angeordnet und mit diesen form- und/oder kraft- und/oder stoffschlüssig verbunden sind.

2. Schuhwerk nach Anspruch 1, wobei das Korrekturband (16) in dem an dem Fuß befestigten Zustand elastisch verformt ist, insbesondere um wenigstens 3 mm elastisch verformt ist.

3. Schuhwerk nach Anspruch 1 oder 2, das derart eingerichtet ist, dass in dem an dem Fuß befestigten Zustand die erste Korrekturkraft (F1) und/oder die zweite Korrekturkraft (F2) erzeugt sind/ist durch eine in dem Korrekturband (16) vorherrschende und durch elastische Verformung bewirkte Spannkraft.

4. Schuhwerk nach einem der Ansprüche 1 bis 3, wobei das Korrekturband (16) in dem an dem Fuß befestigten Zustand des Schuhwerks (10) entlang einer Wirkfläche an der Zehe und/oder dem Zehengrundgelenk anliegt und das Korrekturband (16) entlang seiner Wirkfläche und/oder entlang zu der Wirkfläche angrenzenden Abschnitten elastisch verformt ist.

5. Schuhwerk nach Anspruch 1 bis 4, wobei das Korrekturband (16) in dem befestigten Zustand des Schuhwerks die Zehe zumindest abschnittsweise derart umgreift, dass das Korrekturband (16) sich von einer Seite der Zehe zu der entgegengesetzten Seite der Zehe oder des Zehengrundgelenks erstreckt.

6. Schuhwerk nach einem der Ansprüche 1 bis 5, wobei in dem am Fuß befestigten Zustand das Korrekturband (16) derart an dem Fuß anliegt, dass sich das Korrekturband (16) von einer lateralen Seite der Zehe entlang einer Zehenoberfläche zu einer medialen Seite des Fußes im Bereich des Zehengrundgelenks erstreckt.

7. Schuhwerk nach einem der Ansprüche 1 bis 6, wobei das Korrekturband (16) einen ersten Endabschnitt (22) und einen dazu entgegengesetzt angeordneten zweiten Endabschnitt (24) umfasst, und das Schuhwerk (10) derart ausgestaltet ist, dass in dem am Fuß befestigten Zustand der erste Endabschnitt (22) zwischen der Zehe und einer dazu benachbarten weiteren Zehe positioniert ist und der zweite Endabschnitt (24) im Bereich des Zehengrundgelenks positioniert ist.

8. Schuhwerk nach Anspruch 7, wobei der zweite Endabschnitt (24) seitlich an dem Zehengrundgelenk anliegt oder in einer von dem Zehengrundgelenk zu der Zehe zeigenden Richtung (X) vor oder nach dem Zehengrundgelenk angeordnet ist.

9. Schuhwerk nach einem der Ansprüche 7 oder 8, wobei ein Verbindungsabschnitt (26) des Korrekturbandes (16), über den der erste Endabschnitt (22) des Korrekturbandes (16) mit der Sohle (12) verbunden ist, beabstandet angeordnet ist zu einem proximalen Ende eines Zehenzwischenraums zwischen der Zehe und der benachbarten weiteren Zehe, wobei ein Abstand zwischen dem Verbindungsabschnitt (26) und dem proximalen Ende des Zehenzwischenraums entlang einer Fußlängsachse (X) in einem Bereich zwischen 4 mm und 18 mm liegt.

10. Schuhwerk nach einem der Ansprüche 1 bis 9, wobei das Halteband (18) in dem an dem Fuß befestigten Zustand an oder im Bereich der weiteren Zehe und/oder an oder im Bereich des weiteren Zehengrundgelenks anliegt.

11. Schuhwerk nach einem der Ansprüche 1 bis 10, wobei das Halteband (18) ein elastisches Halteband ist, das in dem an dem Fuß befestigten Zustand elastisch verformt ist, insbesondere um wenigstens 3 mm elastisch verformt ist.

12. Schuhwerk nach einem der Ansprüche 1 bis 11, wobei das weitere Halteband (20) durch ein im Wesentliches zugstarres Halteband gebildet ist.

## Claims

1. Footwear (10) for treating and preventing foot malpositions, in particular for treatment and prevention of hallux valgus, comprising
- a sole (12) comprising an outsole and an insole;
- a holding element (14) configured for securing a foot relative to the footwear (10); and
- a tensioning element (16) configured to be fastened to a toe and configured, in a state in which the footwear (10) is fastened to the foot, to exert a first corrective force (F1) on the toe and to exert a second corrective force (F2) directed opposite to the first corrective force (F1) on a metatarsophalangeal joint of the toe, wherein
the tensioning element (16) is an elastic correcting strap, wherein
the holding element (14) comprises a holding strap (18) and a further holding strap (20), wherein
the holding strap (14) is configured to, in the state in which the footwear (10) is fastened to the foot, secure at least one further toe being arranged adjacent to the toe and/or a further metatarsophalangeal joint relative to the footwear, and
the further holding strap, in the state in which the footwear is fastened to the foot, secures a metatarsal region of the foot relative to the footwear (10) and lies against the foot in the region of its instep, and wherein
oppositely arranged end sections (22, 24, 32, 34, 36) of the correcting strap (16), the holding strap (18) and the further holding strap (20) are guided into the sole (12) and arranged between the outsole and the insole and connected thereto in a form-fittingly and/or force-fittingly and/or adhesively manner.

2. Footwear according to claim 1, wherein in the state fastened to the foot, the correction strap (16) is elastically deformed to an extent of at least 3 mm.

3. Footwear according to claim 1 or 2, which is configured such that, in the state fastened to the foot, the first corrective force (F1) and/or the second corrective force (F2) are/is generated by a tensioning force prevailing in and induced by elastic deformation of the correcting strap (16).

4. Footwear according to any one of claims 1 to 3, wherein in the state of the footwear (10) fastened to the foot, the correcting strap (16), along an engaging surface thereof, lies against the toe and/or the metatarsophalangeal joint, and the correcting strap (16) is elastically deformed along its engaging surface and/or along portions adjacent to the engaging surface.

5. Footwear according to any one of claims 1 to 4, wherein in the state fastened to the foot, the correcting strap (16), at least in sections, engages around the toe such that the correcting strap (16) extends from one side of the toe to an opposite side of the toe or the metatarsophalangeal joint.

6. Footwear according to any one of claims 1 to 5, wherein in the state fastened to the foot, the correcting strap (16) lies against the foot such that the correcting strap (16) extends from a laterale side of the toe along a toe surface to a medial side of the foot in the region of the metatarsophalangeal joint.

7. Footwear according to any one of claims 1 to 6, wherein the correcting strap (16) comprises a first end portion (22) and second end portion (24) opposite arranged thereto, and the footwear (10) is designed such that, in the state fastened to the foot, the first end portion (22) is positioned between the toe and an adjacent further toe and the second end portion (24) is positioned in the region of the metatarsophalangeal joint.

8. Footwear according to claim 7, wherein the second end portion (24) lies sideways against the metatarsophalangeal joint or is arranged in front of or after the metatarsophalangeal joint in a direction (X) pointing from the metatarsophalangeal joint to the toe.

9. Footwear according to claim 7 or 8, wherein a connecting portion (26) of the correcting strap (16), via which the first end portion (22) of the correcting strap (16) is connected to the sole (12), is arranged spaced apart from a proximal end of a toe interspace between the toe and the adjacent further toe, wherein a distance between the connecting portion (26) and the proximal end of the toe interspace along a longitudinal foot axis (X) lies in the range between 4 mm and 18 mm.

10. Footwear according to any one of claims 1 to 9, wherein in the state fastened to the foot, the holding strap (18) lies against or is arranged in the region of the further toe and/or lies against or is arranged in the region of the further metatarsophalangeal joint.

11. Footwear according to any one of claims 1 to 10, wherein the holding strap (18) is an elastic holding strap which, in the state of the footwear fastened to the foot, is elastically deformed to an extent of at least 3 mm.

12. Footwear according to any one of claims 1 to 11, wherein the further holding strap (20) is formed by a substantially tensile rigid holding strap.

## Revendications

1. Chaussure (10) pour le traitement et la prévention de déformations du pied, en particulier pour le traitement et la prévention de l'hallux valgus, comprenant
- une semelle (12) avec une semelle extérieure et une semelle intérieure ;
- un élément de retenue (14) pour fixer un pied par rapport à la chaussure (10) ;
- un élément de serrage (16) à fixer à un orteil, élément qui est conçu afin d'exercer, dans un état fixé au pied de la chaussure (10), une première force de correction (F1) sur l'orteil et d'exercer une seconde force de correction (F2) dirigée à l'encontre de la première force de correction (F1) sur une articulation métatarsophalangienne, dans laquelle
l'élément de serrage (16) est une bande de correction élastique, dans laquelle
l'élément de retenue (14) comprend une bande de retenue (18) et une autre bande de retenue (20), dans laquelle
la bande de retenue (18) fixe dans un état fixé au pied au moins un autre orteil contigu à l'orteil et/ou une autre articulation métatarsophalangienne par rapport à la chaussure (10) et
la bande de retenue supplémentaire (20) fixe dans l'état fixé au pied une zone du métatarse du pied par rapport à la chaussure (10) et repose contre le pied dans la zone d'un coup de pied, et dans laquelle
des sections d'extrémité (22, 24, 32, 34, 36) disposées à l'opposé de la bande de correction (16), de la bande de retenue (18) et de la bande de retenue supplémentaire (20) sont guidées dans la semelle (12) et disposées entre la semelle extérieure et la semelle intérieure et sont reliées à celles-ci par complémentarité de formes et/ou à force et/ou par complémentarité de matière.

2. Chaussure selon la revendication 1, dans laquelle la bande de correction (16) est élastiquement déformée dans l'état fixé au pied, en particulier est élastiquement déformée d'au moins 3 mm.

3. Chaussure selon la revendication 1 ou 2 qui est conçue de telle manière que la première force de correction (F1) et/ou la seconde force de correction (F2) soient générées dans l'état fixé au pied par une force de serrage dominant dans la bande de correction (16) et provoquée par déformation élastique.

4. Chaussure selon l'une quelconque des revendications 1 à 3, dans laquelle la bande de correction (16) repose dans l'état fixé au pied de la chaussure (10) contre l'orteil et/ou l'articulation métatarsophalangienne le long d'une surface active, et la bande de correction (16) est élastiquement déformée le long de sa surface active et/ou le long de sections contiguës à la surface active.

5. Chaussure selon la revendication 1 à 4, dans laquelle la bande de correction (16) entoure au moins par sections l'orteil dans l'état fixé de la chaussure de telle manière que la bande de correction (16) s'étende d'un côté de l'orteil au côté opposé de l'orteil ou de l'articulation métatarsophalangienne.

6. Chaussure selon l'une quelconque des revendications 1 à 5, dans laquelle la bande de correction (16) repose contre le pied dans l'état fixé au pied de telle manière que la bande de correction (16) s'étende d'un côté latéral de l'orteil le long d'une surface de l'orteil à un côté médian du pied dans la zone de l'articulation métatarsophalangienne.

7. Chaussure selon l'une quelconque des revendications 1 à 6, dans laquelle la bande de correction (16) comprend une première section d'extrémité (22) et une seconde section d'extrémité (24) disposée à l'opposé de celle-ci, et la chaussure (10) est configurée de telle manière que dans l'état fixé au pied, la première section d'extrémité (22) soit positionnée entre l'orteil et un autre orteil contigu à celui-ci et la seconde section d'extrémité (24) soit positionnée dans la zone de l'articulation métatarsophalangienne.

8. Chaussure selon la revendication 7,
dans laquelle la seconde section d'extrémité (24) repose latéralement contre l'articulation métatarsophalangienne ou est disposée dans une direction (X) dirigée de l'articulation métatarsophalangienne à l'orteil avant ou après l'articulation métatarsophalangienne.

9. Chaussure selon l'une quelconque des revendications 7 ou 8,
dans laquelle une section de liaison (26) de la bande de correction (16), par le biais de laquelle la première section d'extrémité (22) de la bande de correction (16) est reliée à la semelle (12), est disposée à distance d'une extrémité proximale d'un espace intermédiaire d'orteil entre l'orteil et l'autre orteil contigu, dans laquelle une distance entre la section de liaison (26) et l'extrémité proximale de l'espace intermédiaire d'orteil se trouve le long d'un axe longitudinal de pied (X) dans une zone comprise entre 4 mm et 18 mm.

10. Chaussure selon l'une quelconque des revendications 1 à 9,
dans laquelle la bande de retenue (18) repose dans l'état fixé au pied contre ou dans la zone de l'autre orteil et/ou contre ou dans la zone de l'autre articulation métatarsophalangienne.

11. Chaussure selon l'une quelconque des revendications 1 à 10,
dans laquelle la bande de retenue (18) est une bande de retenue élastique qui est élastiquement déformée dans l'état fixé au pied, en particulier est élastiquement déformée d'au moins 3 mm.

12. Chaussure selon l'une quelconque des revendications 1 à 11, dans laquelle l'autre bande de retenue (20) est formée par une bande de retenue sensiblement rigide à la traction.
